# FASCICULE DE BREVET EUROPEEN

(11) **EP 0 573 552 B1**
(45) Date de publication et mention de la délivrance du brevet: **12.06.1996**
(21) Numéro de dépôt: 92907258.5
(22) Date de dépôt: 25.02.1992
(51) Int. Cl.: C12N 5/08, C12N 5/02, G01N 33/574, C12Q 1/25, C07K 2/00

(54) **CULTURES DE LIGNEES PERMANENTES DE CELLULES PROMYELOCYTAIRES HUMAINES, ET LEURS UTILISATIONS POUR LE CRIBLAGE DE MOLECULES UTILISABLES NOTAMMENT DANS LE TRAITEMENT DES LEUCEMIES**
Zellzucht von permanenten menschlichen Promyelocytezellinien, und deren Anwendung zum Screening von Molekulen für Behandlung von Leukemia.
CULTURES OF PERMANENT LINES OF HUMAN PROMYELOCYTE CELLS, THEIR USE FOR SCREENING MOLECULES USEFUL PARTICULARLY IN TREATING LEUKEMIAS

(30) Priorité: 25.02.1991 FR 9102229
(43) Date de publication de la demande: 15.12.1993
(73) Titulaire: INSTITUT NATIONAL DE LA SANTE ET DE LA RECHERCHE MEDICALE (INSERM), 75654 Paris Cédex 13 (FR)
(72) Inventeur: LANOTTE, Michel, INSERM U301, F-75010 Paris (FR); BERGER, Roland, INSERM U301, F-75010 Paris (FR)
(74) Mandataire: Desaix, Anne
(86) Numéro de dépôt international: FR9200173
(87) Numéro de publication internationale: WO9214815

(56) Documents cités:
- EP-A- 0 143 850
- BLOOD, vol. 54, no. 3, septembre 1979, New York, NY (US); R. GALLAGHER et al., pp. 713-733
- WPIL, Week 8828, Derwent Publications Ltd., London (GB); AN 88-193409
- BIOSIS DATABASE; M. IMAIZUMI et al., AN 83-109596

## Description

La leucémie aigüe promyélocytaire (ou LAP pour Leucémie Aigüe à Promyélocytes, ou encore leucémie de type M3) est une pathologie bien définie dans le domaine des leucémies, et est cytogénétiquement caractérisée par une translocation t(15;17) (q22; q11-12) (Rowley J.D. et al, Lancet 1 : 549, 1977). Cette translocation pourrait jouer un rôle dans la prolifération cellulaire incontrôlée avec blocage de la maturation cellulaire qui se produit dans les LAP.

Des études in vitro et in vivo ont permis de démontrer que l'acide rétinoïque (ou encore désigné ci-après par RA) arrête le processus de prolifération cellulaire des LAP et induit la maturation morphologique et fonctionnelle des promyélocytes vers le stade des granulocytes.

Cet effet de l'acide rétinoïque sur les promyélocytes suggère que l'expression de certains gènes étroitement impliqués dans la transduction du signal RA, ou dans le processus de maturation des cellules, soit altérée par la translocation sus-mentionnée.

Des études in vitro menées sur des promyélocytes provenant de patients atteints de LAP, ont permis de démontrer que le gène codant pour le récepteur α de l'acide rétinoïque (récepteur RARα) normalement situé sur le chromosome 17 est remanié par translocation avec le locus PML normalement situé sur le chromosome 15 (de Thé et al, Nature, 347, pp 558-561, 1990). En utilisant des sondes correspondant à des segments d'ADN localisés au voisinage des points de cassure de la translocation, des réarrangements génomiques de l'un ou de l'autre locus ont pu être déterminés chez les patients atteints de LAP sus-mentionnés. Par conséquent les gènes RARα et PML sont réarrangés dans les LAP. Ces expériences soutiennent fortement l'implication du récepteur α de l'acide rétinoïque dans les leucémies aigües à promyélocytes.

Compte tenu de l'importance du rôle joué par le RARa dans la pathologie des LAP, il serait particulièrement intéressant de pouvoir disposer de lignées cellulaires constituées de promyélocytes d'origine humaine comportant cette translocation t(15 ; 17) caractéristique des LAP, afin notamment de cribler des molécules dérivées de l'acide rétinoïque, ou autres molécules, susceptibles de restaurer la maturation cellulaire sans pour autant présenter les effets toxiques de l'acide rétinoïque.

Cependant, aucune lignée de cellules promyélocytaires humaines n'a pu être maintenue en culture jusqu'à maintenant et n'a pu par conséquent permettre ce type d'étude.

Bien que la lignée HL60 ait été appelée "promyélocytaire" (Gallagher R. et al, Blood 54 : 713, 1979), il est désormais reconnu d'une part qu'elle provient d'une leucémie myéloblastique caractérisée par des cellules avec un certain degré de maturation (M2) (Dalton WT et al, Blood 71 ; 242, 1988), et d'autre part qu'elle ne contient pas la translocation t(15;17) sus-mentionnée.

Un des buts de la présente invention est précisément de disposer d'une culture cellulaire permettant de cribler des molécules susceptibles de restaurer la maturation cellulaire.

La présente invention a pour objet des lignées cellulaires permanentes, caractérisées en ce qu'elles sont essentiellement constituées de cellules promyélocytaires caractéristiques des leucémies aigües promyélocytaires humaines, ces cellules étant cytogénétiquement caractérisées par une translocation t(15;17), et susceptibles de proliférer indéfiniment dans un milieu de culture.

Le milieu de culture dans lequel sont susceptibles de proliférer les cellules sus-mentionnées, est avantageusement constitué d'un milieu nutritif synthétique (RPMI 1640) et de sérum de veau foetal.

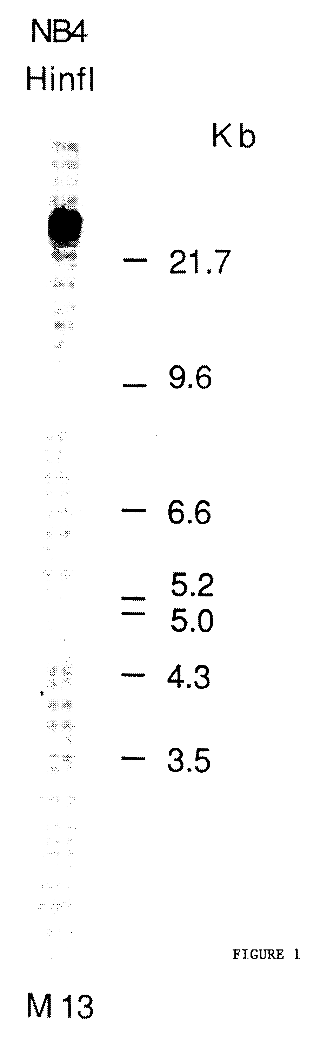

Les lignées cellulaires de l'invention et leurs variantes sont particulièrement caractérisées en ce qu'elles présentent des empreintes génétiques sur gel d'electrophorèse identiques, en totalité ou en partie, à l'empreinte génétique représentée sur la Figure 1.

A titre illustratif, les empreintes génétiques des cellules constituant les lignées cellulaires de l'invention, sont obtenues selon le mode opératoire défini dans Nucleic Acids Research (1988) vol. 16, n°9, p. 4161.

Les lignées cellulaires de l'invention sont davantage caractérisées en ce qu'elles sont soit sensibles à l'acide rétinoïque, c'est à dire susceptibles de se différencier du stade des promyélocytes vers le stade des granulocytes sous l'effet de l'acide rétinoïque, soit, au contraire, résistantes à l'acide rétinoïque, et donc incapables de se différencier sous l'effet de ce dernier, ou encore capables de se différencier par traitement à l'aide d'acide rétinoïque associé à un ou plusieurs agents inducteurs d'une telle différenciation.

L'invention concerne une lignée cellulaire sensible à l'acide rétinoïque (c'est à dire dont la majorité des cellules sont susceptibles de se différencier sous l'effet de ce dernier), et qui a fait l'objet d'un dépôt à la Collection Nationale de Culture de Micro-organismes de l'INSTITUT PASTEUR (CNCM) le 25 février 1991 sous le numéro I-1045, ainsi qu'à la Deutsche Sammlung von Mikroorganismen und ZellKulturen GmbH (DSM) le 14 février 1992 sous le numéro DSM.ACC.2030.

Cette lignée cellulaire a été obtenue à partir d'un patient atteint d'une LAP, traité par chimiothérapie et en phase de rechute. Des cellules de la moëlle osseuse ont été prélevées chez ce patient, et ont été mises en culture sur une couche de cellules du stroma de moëlle osseuse (Lanotte et al J. Cell. Sci. 50 : 281, 1981) jusqu'à l'obtention d'une lignée cellulaire autonome (ci-après désignée par l'expression NB4). Cette lignée cellulaire NB4 est particulièrement caractérisée par l'empreinte génétique représentée sur la Figure 1, et principalement constituée de cellules sensibles à l'acide rétinoique. Elle fait l'objet d'une étude plus approfondie dans la description détaillée de l'invention qui suit.

L'invention a également pour objet une lignée cellulaire résistante à l'acide rétinoique (c'est à dire dont la majorité des cellules sont incapables de se différencier sous l'effet de ce dernier), cette lignée (ci-après désignée par l'expression NB4R) étant obtenue par incubation de la lignée NB4 précédente en présence d'acide rétinoïque tout-trans, suivie de l'isolement des cellules résistantes et de l'amplification de ces dernières toujours en présence d'acide rétinoïque.

L'invention concerne également un procédé de criblage de molécules susceptibles de restaurer la maturation cellulaire, et plus particulièrement d'induire la maturation des promyélocytes vers le stade des granulocytes, notamment dans le cadre des leucémies aigües promyélocytaires humaines, ce procédé étant caractérisé en ce qu'il comprend la mise en contact d'une quantité déterminée de la molécule étudiée avec une lignée cellulaire de l'invention, et plus particulièrement avec une lignée sensible, suivie de la mise en évidence d'une éventuelle différenciation des cellules de ladite lignée.

L'invention a également pour objet un procédé de criblage de molécules susceptibles de lever la résistance que présentent des cellules, et plus particulièrement des cellules caractéristiques des leucémies aigües promyélocytaires humaines, vis à vis de l'effet de différenciation de l'acide rétinoïque ou d'autres composés normalement susceptibles d'induire la maturation cellulaire mais pour lesquels des phénomènes de résistance des cellules concernées peuvent être observés, ledit procédé étant caractérisé en ce qu'il comprend la mise en contact d'une quantité déterminée de la molécule étudiée avec une lignée cellulaire résistante telle que définie ci-dessus, suivie de la mise en évidence d'une éventuelle différenciation des cellules de ladite lignée en présence d'acide rétinoïque ou des autres composés sus-mentionnés.

Le procédé sus-mentionné permet avantageusement de sélectionner des molécules qui soient susceptibles d'une part de lever ce phénomène de résistance dont il est question ci-dessus, et, d'autre part, de restaurer la maturation cellulaire dans les conditions sus-mentionnées.

Dans le cadre des procédés sus-mentionnés, la détection de la différenciation des promyélocytes des lignées cellulaires de l'invention vers le stade des granulocytes est avantageusement réalisée par application de l'une ou plusieurs des méthodes suivantes.

Cette différenciation se traduisant notamment par l'activation de l'expression de certains gènes spécifiques, les produits d'expression de ces gènes sont détectés à l'aide d'anticorps spécifiques, ces anticorps étant le cas échéant marqués notamment de manière radioactive ou enzymatique.

Lorsque ces produits d'expression présentent une activité enzymatique ou lorsque cette maturation se traduit par une disparition d'une activité enzymatique, telle que l'activité myélopéroxydase, la détection de la différenciation est avantageusement réalisée à l'aide de substrats, le cas échéant marqués, notamment de manière radioactive ou enzymatique, et sur lesquels les produits enzymatiquement actifs exprimés, ou qui ne sont plus exprimés, sont susceptibles d'agir.

La détection de cette différenciation cellulaire peut également être réalisée par une étude morphologique des cellules, notamment après coloration de ces dernières. A titre d'exemple de méthode de coloration des cellules, on peut citer la coloration de May-Grünwald-Giemsa.

La différenciation cellulaire peut également s'apprécier selon un critère fonctionnel, c'est à dire que l'on étudie si les cellules différenciées sont fonctionnelles au même titre que des granulocytes normaux. A titre d'exemples de test susceptible de permettre la détection de critères fonctionnels de granulocytes normaux, on citera : le test NBT (Nitro Blue Tetrazolium) (Pick E., Methods Enzymol. 133:407, 1986).

Le temps de réponse aux molécules étudiées des lignées cellulaires de l'invention lors de la mise en oeuvre des procédés décrits ci-dessus, varie de quelques heures à quelques jours en fonction de la méthode de détection de la différenciation cellulaire choisie.

A titre illustratif, le temps d'incubation nécessaire avant détection de l'activation de l'expression de gènes est de l'ordre de 30 minutes à 24 heures, celui nécessaire avant détection de la modification de l'aspect morphologique des cellules est de 15 heures à 5 jours, et celui nécessaire avant détection de l'aspect fonctionnel des cellules est de 3 jours à 7 jours.

De manière avantageuse, ces méthodes de criblage permettent de sélectionner des molécules susceptibles d'être utilisées dans le domaine de la prévention ou du traitement de pathologies causées par une prolifération anormale des cellules, notamment des tumeurs malignes en général, et plus particulièrement des leucémies, notamment les leucémies aigües promyélocytaires.

Ces méthodes permettent plus particulièrement dans le cas de l'utilisation de lignées cellulaires résistantes à l'acide rétinoïque, de sélectionner des molécules susceptibles d'être utilisées dans le cadre de la prévention de l'apparition de phénomènes de résistance vis à vis de l'effet de différenciation de l'acide rétinoïque ou d'autres composés normalement susceptibles d'induire la maturation cellulaire, des cellules d'un malade lors d'un traitement de ce dernier avec l'acide rétinoïque ou des composés sus-mentionnés.

Toujours dans le cas de l'utilisation de lignées cellulaires résistantes à l'acide rétinoïque, ces procédés selon l'invention permettent de sélectionner des molécules susceptibles d'être utilisées dans le cadre du traitement de patients en rechute après traitement avec l'acide rétinoïque ou d'autres composés normalement susceptibles d'induire la maturation cellulaire.

L'invention vise également les cultures elles-mêmes constitutées par des cellules des lignées cellulaires de l'invention, notamment des cellules NB4 ou NB4R sus-mentionnées, et d'un support nutritif permettant la prolifération des cellules, notamment le milieu RPMI 1640 additionné de sérum de veau foetal indiqué plus haut.

A ce titre l'invention a pour objet des kits pour la mise en oeuvre des procédés de criblage tels que décrits ci-dessus, ces kits comprenant :
- une culture telle que décrite ci-dessus comprenant une lignée cellulaire selon l'invention,
- des réactifs appropriés pour permettre la détection de l'éventuelle différenciation des cellules, notamment :
   * des anticorps susceptibles de reconnaître spécifiquement les polypeptides produits par l'activation de l'expression de certains gènes, ces anticorps étant le cas échéant marqués notamment de manière radioactive ou enzymatique,
   * des substrats, le cas échéant marqués, notamment de manière radioactive ou enzymatique, et sur lesquels les produits enzymatiquement actifs exprimés, ou qui ne sont plus exprimés, sont susceptibles d'agir,
   * des enzymes, le cas échéant marqués, notamment de manière radioactive ou enzymatique, et susceptibles d'agir sur les substrats produits lors de la différenciation cellulaire,
   * des colorants cellulaires.

Les lignées cellulaires de l'invention sont également caractérisées en ce que les surfaces membranaires des cellules les constituant sont notamment reconnues par la totalité des anticorps respectivement spécifiquement dirigés contre des antigènes de membranes de leucocytes humains de classe I (HLA class I), les marqueurs CD13 et CD33 des cellules myéloïdes, les marqueurs CD15 et CDllb des granulocytes, les marqueurs CD9 et CD11b des monocytes, le marqueur CDllb de l'α-intégrine, le marqueur CD38 des cellules T activées, le marqueur CD2 des cellules T, le marqueur CD4 du récepteur HIV des cellules T helper,
ou sont reconnues par certains d'entre eux seulement,
mais ne sont pas reconnues par les anticorps dirigés contre les antigènes de membranes de leucocytes humains de classe II (HLA class II), le marqueur CD10 des antigènes de membrane de la surface des leucocytes des leucémies aigües lymphocytaires (CALLA) , les marqueurs CD11c, CD14, CD36 des monocytes, le marqueur CD11c de l'α-intégrine et des granulocytes, le marqueur CD36 des plaquettes, le marqueur CD7 des cellules T immatures, le marqueur CD3 des cellules T, le récepteur cellulaire de l'IL-2 chaîne β, le marqueur CD19 des cellules B Pan, le marqueur CD23 des cellules B matures, le marqueur CD34 des cellules précurseurs des lymphocytes et des myélocytes, le marqueur CD41 des plaquettes du groupe II/IIIa, le marqueur CD42 des plaquettes du groupe IX, les érythrocytes, la glycophorine, ou ne sont pas reconnues par certains d'entre eux seulement.

La présence du marqueur CD4 à la surface des cellules de l'invention confère à ces dernières la propriété d'être un modèle de choix pour l'étude du processus de l'infection des cellules par les virus du type HIV.

La présente invention a également pour objet l'utilisation des lignées cellulaires sus-mentionnées en tant que témoins de controle positif dans le cadre de procédés de diagnostic in vitro des leucémies aigües promyélocytaires humaines, ou encore l'utilisation des lignées cellulaires résistantes décrites ci-dessus en tant que témoins de controle positif dans le cadre de procédés de diagnostic in vitro de l'apparition éventuelle de cellules résistantes à l'acide rétinoïque ou autres composés susceptibles d'induire la maturation cellulaire lors du traitement d'un patient avec ces composés.

L'invention vise également des kits pour la mise en oeuvre de procédés de diagnostic in vitro des leucémies aigües promyélocytaires humaines, ces kits comprenant une lignée cellulaire choisie parmi celles décrites ci-dessus en tant que témoin de controle positif de la présence de cellules caractéristiques des leucémies aigües promyélocytaires humaines.

L'invention concerne également ou encore des kits pour la mise en oeuvre de procédés de diagnostic in vitro de l'apparition éventuelle de cellules résistantes à l'acide rétinoïque ou autres composés susceptibles d'induire la maturation cellulaire lors du traitement d'un patient avec ces composés, ces kits comprenant une lignée cellulaire résistante choisie parmi celles décrites ci-dessus en tant que témoin de controle positif de la présence de cellules résistantes aux composés sus-mentionnés.

La présente invention a également pour objet des polypeptides produits par les lignées cellulaires de l'invention, qu'ils soient secrétés ou non, et impliqués dans le mécanisme de prolifération autonome des cellules de ces lignées.

Ces polypeptides, par exemple des facteurs de croissance, sont obtenus à partir des lignées cellulaires de l'invention, notamment selon la méthode comprenant les étapes suivantes :
- incubation des cellules des lignées de l'invention dans un milieu de culture,
- prélèvement du milieu de sécrétion de ces cellules et détection de la présence éventuelle d'un ou plusieurs polypeptides tels que définis ci-dessus à l'aide d'un test de prolifération et de différenciation réalisé sur des cellules hématopoïétiques de référence, ou sur les lignées NB4 et NB4R ou leurs variantes,
- séparation biochimique (chromatographie, électrophorèse etc...) du ou des polypeptides sus-mentionnés.

Ces étapes décrites ci-dessus permettent d'obtenir la ou les molécule(s) naturelle(s).

L'obtention de la (ou des) molécule(s) recombinante(s) peut être réalisée par criblage d'une banque d'expression des lignées NB4 et NB4 ou d'une variante de ces lignées productrice de l'activité biologique recherchée, suivie du clonage génétique et de la production de la (ou des) molécule(s) recombinante(s) sus-mentionnée(s).

L'invention a plus particulièrement pour objet le ou les facteurs de croissance issus des lignées cellulaires NB4 et NB4R, et susceptibles d'être obtenus selon le mode opératoire indiqué ci-dessus.

L'invention sera plus particulièrement illustrée à l'aide de la description détaillée qui suit de l'obtention de la lignée cellulaire NB4 et de la lignée NB4R.

### I MATERIELS ET METHODES

### a) Culture cellulaire et établissement de la lignée cellulaire NB4

Un échantillon de moelle a été obtenu à partir d'un patient atteint de LAP après que ce patient ait reçu un traitement par l'acide rétinoïque. Les leucocytes ont été séparés par centrifugation sur Ficoll-Hypaque et cultivés à une concentration de 10⁴ cellules/ml sur un support nutritif constitué de cellules du stroma de moëlle osseuse humaine (Lanotte et al, J. Cell Sci 50:281, 1981), avec un milieu RPMI 1640 (GIBCO, Grand Island, NY) complété avec 12,5 % de sérum de veau foetal (SVF), 7,5 % de sérum de cheval (Flow Lab, Scotland), dans un flacon en plastique à 37°C dans un air humidifié plus 5 % de CO₂. Les cultures ont été entretenues chaque semaine en remplaçant une moitié du milieu de croissance avec du milieu frais. Après 4 à 5 semaines, la couche adhérente contenant des régions de cellules leucémiques proliférantes a été détachée mécaniquement et la population cellulaire entière a été transférée sans dissociation dans un flacon contenant un support nutritif frais constitué de cellules de stroma de moëlle osseuse. De cette manière un enrichissement progressif de cellules LAP proliférantes a été obtenu. Un développement dépendant du microenvironnement a été observé pendant quatorze semaines. Une croissance autonome a alors été détectée et une population cellulaire se développant rapidement a envahi la culture et est devenue une lignée cellulaire. Les cellules LAP ainsi obtenues sont désignées NB4. Les conditions de croissance NB4 ont été améliorées en cultivant les cellules à une concentration de 2x10⁵ cellules/ml dans un milieu RPMI 1640 complété par 10 % de SVF seul (le temps de doublement est de 36 à 40 heures). Des quantités aliquotes de cellules ont été congelées à -80°C.

### b) Analyse cytogénétique

Les chromosomes ont été étudiés lorsque la lignée cellulaire a été établie et après 40 semaines de culture avec 2 passages par semaine. La technique de bandes R (RHG) a été utilisée et les chromosomes ont été classés selon la nomenclature internationale (ISCN [1985]: An International System for Human Cytogenetic Nomenclature) (Harnden DG, Klinger HP [eds] Published in collaboration with Cytogenet Cell Genet. Basel, Switzerland, Karger, 1985).

### c) Induction de la différenciation

Les cellules NB4 (10⁵ cellules/ml), dans du milieu RPMI contenant 10 % de SVF et des concentrations variées d'acides tout-transrétinoïques (Sigma) en tant qu'agent inducteur, ont été incubées pendant 1 à 5 jours dans des microplaques de culture. La maturation a été évaluée par examen microscopique de la coloration enzymatique sur des plaques cytologiques et aussi par l'adhésion à la matrice extracellulaire des cellules du stroma, à la fibronectine. Les cellules ont été colorées à l'aide de May-Grünwald-Giemsa. Les réactions de la myélopéroxydase, de l'α-naphtyl butyrate estérase, de la naphtol-ASD chloroacétate estérase, de la phosphatase alcaline et au NBT ont été effectuées.

### d) Marqueurs de surface cellulaire

La coloration pour l'immunofluorescence indirecte sur les suspensions cellulaires a été réalisée selon les méthodes décrites dans (Chen Z. et al. : Immunological typing of ALL: Concurrent analysis by flow cytometry and immunocytology. Leuk Res 10:1411, 1986). L'analyse cytofluométrique a été réalisée sur un profil EPICS (Coultronics, Margency, France). Un grand nombre d'anticorps monoclonaux (cf. supra) a été utilisé.

### II RESULTATS

### a) Etablissement, morphologie et cytométrie de la lignée cellulaire

Les cellules LAP ont été cultivées sur une couche de cellules du stroma de moëlle. Au début de la culture, une sous-population de cellules ayant réalisé une certaine "coopération" avec le microenvironnement hématopoïétique a été sélectionnée. La population globale, avec des pertes d'interaction avec les cellules du stroma était constituée de promyélocytes hypergranulaires en phase G0/G1, tandis que quelques cellules leucémiques fortement associées aux cellules du stroma, avaient une activité mitotique, étaient beaucoup moins granulaires, avec la morphologie des cellules blastiques trouvées dans les LAP. Au moment de l'isolement de la lignée cellulaire NB4, seules les cellules blastiques prolifèrent. Après 9 mois de culture (soit environ 155 doublements de cellules) aucun changement significatif n'a été constaté.

### b) Etude cytogénétique

Les analyses cytogénétiques ont été réalisées au stade initial de la culture au moment de l'isolement de la lignée cellulaire NB4, et après neuf mois de culture. Au stade initial de la culture, les 23 métaphases examinées étaient anormales avec des variations caryotypiques d'une cellule à l'autre. Leur nombre de chromosomes variait entre 68 et 90, la plupart d'entre eux se situant dans le domaine hypotétraploïde, avec des pertes au hasard.

Toutes les métaphases ont la translocation t(15;17)(q22;q11-12) associée avec d'autres remaniements variables d'une cellule à l'autre. Toutefois, une perte du chromosome 19 et un remplacement par 19q+ d'une part, et un der(12;?) (p12;?) était présent sur toutes les métaphases examinées.

La translocation t(15;17) présente dans le premier caryotype réalisé à partir du patient sus-mentionné atteint de LAP et au stade de sa seconde rechute, a été observée dans toutes les métaphases de la lignée cellulaire établie. La complexité caryotypique qui a été observée dans la culture de moëlle osseuse à court terme a été retrouvée dans la lignée cellulaire avec une grande variation d'une cellule à une autre. Le caryotype était hypotétraploïde avec perte au moins d'une copie des chromosomes 8, 11 et 14. Un caryotype repésentatif peut être résumé de la manière suivante :
80-87,XXX,-X,-3,-8,-9,-10,-12,-14,-14,-18,-19,-19, der(12),t(12;?)(p12;),t(15;17)(q22;q11-12),t(15;17), der(19)t(19;?), + marqueurs variables.

En raison de cette variation, il est difficile d'établir les différences mineures entre les caryotypes des cellules "fraîches" et les caryotypes des cellules de la lignée NB4. Toutefois, un nouveau marqueur der(12) a pu être détecté dans la lignée cellulaire alors qu'il n'était pas présent dans les cellules "fraîches". Le remaniement du chromosome 19, ressemblant à HSR(19)(q13), que l'on trouve dans certaines métaphases des cellules "fraîches", a apparemment été selectionné durant l'établissement de la lignée cellulaire car il était présent dans toutes les métaphases, la plupart du temps dupliqué.

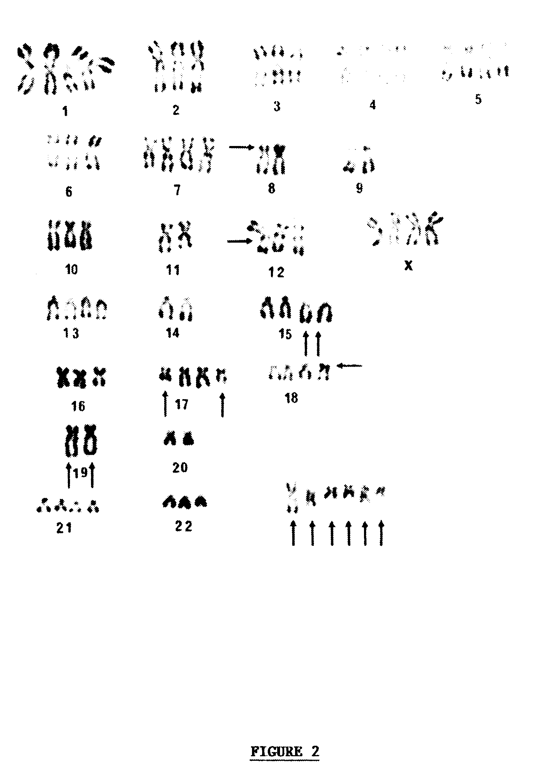

Le caryotype de la lignée NB4 est représenté sur la Figure 2.

### c) Analyse immunocytologique

Les marqueurs de surface cellulaire de NB4 ont été analysés (cf. supra) et suivis pendant 9 mois, mais aucun changement significatif n'a été détecté. Il a ainsi été déterminé que NB4 exprime des marqueurs spécifiques du stade granulocytaire, mais également des marqueurs de cellules lymphoïdes telles que CD2, CD4, et un marqueur des monocytes CD9. Les pourcentages des cellules positives vis-à-vis des marqueurs myéloïdes (73 % à 89 %) et des marqueurs des cellules T (73 % pour CD4) suggèrent que les caractéristiques des deux lignées sont simultanément exprimées. CD9 est exprimée par 73 % des cellules, tandis que les cellules sont clairement négatives pour ce qui concerne les autres marqueurs liés au monocyte tels que CD14 ou CD36. L'expression de ces antigènes pourrait être reliée à des activations de gènes liés aux altérations de caryotypes multiples qui ont été trouvées.

### d) L'acide rétinoïque induit la maturation

Des cellules NB4 ont été traitées avec de l'acide rétinoïque trans (1µmol/l) pendant six jours. L'arrêt de la prolifération cellulaire est survenu après 48 heures ; des maturations morphologiques accompagnées de modifications des antigènes de surface et des marqueurs fonctionnels peuvent être détectées. Une forte augmentation du marqueur corrélé à l'α-intégrine, CD11b (Arnaout MA: Structure and function of the leucocyte adhesion molecules CD11 CD18. Blood 75:1037, 1990) a été remarquée ; CD11c, qui est absent des cellules blastiques est fortement exprimé sur 75 % des cellules différenciées ; ces changements sont associés avec une augmentation marquée de l'adhésion des cellules aux matrices extracellulaires. La production de superoxyde et peroxyde d'hydrogène, et la réduction du NBT ont été quantitativement analysées par ELISA et par des méthodes cytochimiques ; une forte augmentation de la positivité du test NBT indique une capacité potentielle antimicrobienne corroborant la maturation morphologique induite par l'acide rétinoïque.

### III ANALYSE DES RESULTATS

La lignée cellulaire NB4 est la seule lignée permanente présentant une translocation t(15;17) établie à partir de cellules leucémiques d'un patient atteint de LAP.

Les cellules provenant de LAP humaines (M3) présentent une manière singulière de proliférer in vitro qui est la cause probable des échecs réalisés jusqu'à ce jour afin d'obtenir une lignée cellulaire permanente. Il peut être conclu de ces échecs : (1) que les cellules LAP ont un potentiel de prolifération in vitro très faible. L'analyse du cycle cellulaire par cytométrie démontre un arrêt de la prolifération en phase G1 (ou éventuellement en phase G0) durant plusieurs semaines et pendant lesquelles les cellules deviennent hypergranulaires et présentent une réaction myélopéroxydase intense. Toutefois, les cellules de LAP survivent en culture, alors que beaucoup de types cellulaires de leucémies meurent ; cela suggère que ces cellules ne requièrent pas de facteur de survie, à la différence des promyélocytes normaux ou des lignées cellulaires dépendantes de facteurs. (2) aucun des facteurs de croissance hématopoïétique testés jusqu'à maintenant (y compris G-CSF) ne permet la prolifération in vitro sur plus de 3 à 4 cycles cellulaires. (3) les cellules de LAP réalisent des interactions cellules-cellules ou cellules-matrices avec les cellules du stroma de la moelle osseuse. Quelques cellules de LAP associées au microenvironnement (1 à 10 pour 10⁵ cellules) prolifèrent.

Compte tenu de ce qui précède on pourrait s'attendre à ce que des cellules de LAP de stroma de moëlle cocultivée pendant plusieurs mois pourraient permettre la selection de cellules capables de proliférer. Les auteurs de la présente demande ont émis l'hypothèse que l'isolement d'une lignée cellulaire de LAP permanente dépend d'un second évènement responsable de l'autonomie de prolifération et que sa probabilité devrait considérablement augmentée dans une population en cycle par comparaison avec une population arrêtée au stade G1. Cette stratégie a été adoptée pour isoler la lignée cellulaire NB4. La culture primaire de cellules leucémiques sur une couche de cellules de stroma fournit un environnement favorable pour la prolifération cellulaire. Il ne peut être affirmé qu'une mutation additionnelle est responsable de l'apparition de cellules leucémiques autonomes qui se développent dans la culture de cellules de LAP dépendant du microenvironnement. Il faut souligner qu'un marqueur additionnel der(12) est présent dans la lignée cellulaire mais ne peut être détecté dans les cellules "fraîches". Par contre une population cellulaire s'autorenouvelant pourrait être déjà présente dans la moëlle du patient et aurait pu être sélectionnée in vitro. Plusieurs observations jouent en faveur de cette dernière hypothèse : (1) Parmi les multiples changements de chromosomes dans les cellules blastiques du patient, qui sont peut-être partiellement reliés à des traitements antérieurs, quelques remaniements favorisant la prolifération in vitro peuvent avoir été selectionnés (2) Les cellules NB4 ont conservé des caractéristiques morphologiques d'une population minoritaire de cellules blastiques qui a été également trouvée chez le patient au moment de sa rechute.

L'acide rétinoïque induit une maturation rapide des cellules NB4 du point de vue morphologique et fonctionnelle ; la prolifération cellulaire n'est plus détectable après 3 jours d'un traitement continu à l'acide rétinoïque. La maturation des cellules NB4 avec d'autres inducteurs tels que les esters de phorbol, le diméthyl-sulfoxide, les nucléotides cycliques, et les corticostéroïdes, n'égale jamais tant en intensité qu'en rapidité la maturation induite par l'acide rétinoïque.

La figure 1 représente l'empreinte génétique de la lignée cellulaire NB4, et qui est obtenue par digestion du génome des cellules avec Hinf I, suivie d'une migration des fragments d'ADN obtenus sur gel d'électrophorèse et détection de fragments à l'aide de la sonde M13 (cf. Nucleic Acids Research sus-mentionné).

La Figure 2 représente le caryotype de la lignée cellulaire NB4, les flèches indiquent les chromosomes remaniés.

### IV OBTENTION DE LA LIGNEE NB4R

Des cellules de l'isolat initial de la lignée promyélocytaire humaine NB4 ont été incubées en présence constante d'acide rétinoïque tout-trans: après plusieurs mois de culture, quelques cellules résistantes ont été isolées et amplifiées toujours en présence d'acide rétinoïque. Cet isolat a permis d'isoler une lignée NB4 résistante à l'acide rétinoïque (lignée NB4R). Ces cellules résistantes constituent une lignée autonome, permanente au même titre que la lignée NB4 sensible et peuvent être maintenues dans les mêmes conditions. Il est intéressant de noter que cette résistance correspond à un fait clinique couramment observé, à savoir la rechute après une rémission plus ou moins prolongée consécutive au traitement par l'acide rétinoïque. Le malade dont sont issues ces lignées était lui-même en rechute après chimiothérapie et traitement par l'acide rétinoïque. On peut donc dire que les deux isolats NB4 et NB4R, l'un sensible à l'acide rétinoïque, l'autre résistant, représentent deux volets de cette pathologie. Ces cellules permettent d'étudier le mécanisme d'acquisition de la résistance au traitement des leucémies promyélocytaires par l'acide rétinoïque. Cette complémentarité renforce l'intérêt de l'outil que constituent ces lignées pour l'étude des effets des agents thérapeutiques et pour la recherche des moyens de prévenir (ou de lever) la résistance à ces agents. La lignée résistante constitue le seul modèle biologique existant d'une lignée promyélocytaire avec t(15;17) permettant de réaliser des criblages moléculaires en vue de découvrir de nouveaux médicaments actifs sur les cellules de malades en rechute après traitement avec l'acide rétinoïque.

## Revendications

1. Lignée cellulaire permanente, caractérisée en ce qu'elle est essentiellement constituée de cellules promyélocytaires humaines caractéristiques des leucémies aigües promyélocytaires, ces cellules étant cytogénétiquement caractérisées par une translocation t(15;17), susceptibles de proliférer indéfiniment dans un milieu de culture synthétique contenant du sérum de veau foetal.

2. Lignée cellulaire selon la revendication 1, caractérisée en ce que les cellules la constituant présentent des empreintes génétiques sur gel d'electrophorèse identiques, en totalité ou en partie, à l'empreinte génétique représentée sur la Figure 1.

3. Lignée cellulaire selon la revendication 1 ou la revendication 2, caractérisée en ce qu'elle est sensible à l'acide rétinoique.

4. Lignée cellulaire selon l'une des revendications 1 à 3, déposée à -la CNCM le 25 février 1991 sous le numéro I-1045, ainsi qu'à la Deutsche Sammlung von Mikroorganismen und ZellKulturen GmbH (DSM) le 14 février 1992 sous le numéro DSM.ACC.2030.

5. Lignée cellulaire selon la revendication 1 ou la revendication 2, caractérisée en ce qu'elle est résistante à l'acide rétinoïque.

6. Lignée cellulaire selon la revendication 5 telle qu'obtenue par incubation de la lignée selon la revendication 4 en présence d'acide rétinoique tout-trans, suivie de l'isolement des cellules résistantes et de l'amplification de ces dernières toujours en présence d'acide rétinoïque.

7. Culture cellulaire essentiellement constituée de cellules appartenant à une lignée cellulaire telle que définie dans l'une des revendications 1 à 6, en association avec des éléments nutritifs permettant la prolifération des cellules.

8. Procédé de criblage de molécules susceptibles de restaurer la maturation cellulaire, caractérisé en ce qu'il comprend la mise en contact d'une quantité déterminée de la molécule étudiée avec une lignée cellulaire selon l'une des revendications 1 à 4, suivie de la mise en évidence d'une éventuelle différenciation des cellules de ladite lignée.

9. Procédé de criblage de molécules susceptibles de lever la résistance que présentent des cellules, et plus particulièrement des cellules caractéristiques des leucémies aigües promyélocytaires humaines, vis à vis de l'effet de différenciation de l'acide rétinoïque ou d'autres composés normalement susceptibles d'induire la maturation cellulaire mais pour lesquels des phénomènes de résistance des cellules concernées peuvent être observés, ledit procédé étant caractérisé en ce qu'il comprend la mise en contact d'une quantité déterminée de la molécule étudiée avec une lignée cellulaire selon la revendication 5 ou la revendication 6, suvie de la mise en évidence d'une éventuelle différenciation des cellules de ladite lignée en présence d'acide rétinoïque ou des autres composés sus-mentionnés.

10. Procédé selon la revendication 8 ou la revendication 9, caractérisé en ce que la mise en évidence de la différenciation des cellules est réalisée :
- par détection des produits d'expression de gènes spécifiquement exprimés au cours de la différenciation, à l'aide d'anticorps susceptibles de reconnaître spécifiquement les polypeptides produits, ces anticorps étant le cas échéant marqués notamment de manière radioactive ou enzymatique, ou à l'aide de substrats, le cas échéant marqués, notamment de manière radioactive ou enzymatique lorsque ces produits d'expression sont enzymatiquement actifs,
- ou par étude morphologique des cellules,
- ou encore par étude fonctionnelle des cellules.

11. Kit pour la mise en oeuvre d'un procédé selon l'une des revendications 8 à 10, caractérisé en ce qu'il comprend :
- une lignée cellulaire selon l'une des revendications 1 à 6,
- des réactifs appropriés pour permettre la détection de l'éventuelle différenciation des cellules, notamment :
* des anticorps susceptibles de reconnaître spécifiquement les polypeptides produits par l'activation de l'expression de certains gènes, ces anticorps étant le cas échéant marqués notamment de manière radioactive ou enzymatique,
* des substrats, le cas échéant marqués, notamment de manière radioactive ou enzymatique, et sur lesquels les produits enzymatiquement actifs exprimés, ou qui ne sont plus exprimés, sont susceptibles d'agir,
* des enzymes, le cas échéant marqués, notamment de manière radioactive ou enzymatique, et susceptibles d'agir sur les substrats produits lors de la différenciation cellulaire,
* des colorants cellulaires.

12. Utilisation d'une lignée cellulaire selon l'une des revendications 1 à 6, en tant que témoin de controle positif dans le cadre de procédés de diagnostic in vitro des leucémies aigües promyélocytaires humaines, ou encore de procédés de diagnostic in vitro ou encore de procédés de diagnostic in vitro de l'apparition éventuelle de cellules résistantes à l'acide rétinoïque ou autres composés susceptibles d'induire la maturation cellulaire lors du traitement d'un patient avec ces composés.

13. Kits pour la mise en oeuvre de procédés de diagnostic in vitro des leucémies aigües promyélocytaires humaines, ou encore de procédés de diagnostic in vitro de l'apparition éventuelle de cellules résistantes à l'acide rétinoïque ou autres composés susceptibles d'induire la maturation cellulaire lors du traitement d'un patient avec ces composés, ces kits comprenant une lignée cellulaire selon l'une des revendications 1 à 6 en tant que témoin de controle positif de la présence de cellules caractéristiques des leucémies aigües promyélocytaires humaines, et, le cas échéant de cellules résistantes aux composés sus-mentionnés.

14. Procédé d'obtention d'un facteur de croissance, caractérisé en ce qu'il comprend les étapes suivantes :
- incubation des cellules des lignées selon l'une quelconque des revendications 1 à 6 dans un milieu de culture,
- prélèvement du milieu de sécrétion de ces cellules et détection de la présence éventuelle d'un ou plusieurs polypeptides tels que définis ci-dessus à l'aide d'un test de prolifération et de différenciation réalisé sur des cellules hématopoïétiques de référence, ou sur la lignée selon la revendication 4 et la lignée selon la revendication 4 résistante à l'aide rétinoïque ou leurs variantes,
- séparation biochimique du ou des polypeptides sus-mentionnés.

## Patentansprüche

1. Permanente Zellinie,
dadurch gekennzeichnet, daß sie im wesentlichen aus menschlichen Promyelozytenzellen besteht, die für akute promyelozytäre Leukämien charakteristisch sind, wobei diese Zellen cytogenetisch durch eine Translokation t(15;17) gekennzeichnet und in der Lage sind, unbegrenzt in einem synthetischen Kulturmedium, das fötales Kälberserum enthält, zu proliferieren.

2. Zellinie nach Anspruch 1,
dadurch gekennzeichnet, daß die Zellen, aus denen sie besteht, auf einem einer Elektrophorese unterworfenen Gel vollständig oder teilweise hinsichtlich des in Figur 1 gezeigten genetischen Fingerprints identische genetische Fingerprints aufweisen.

3. Zellinie nach Anspruch 1 oder Anspruch 2,
dadurch gekennzeichnet, daß sie gegenüber Retinsäure empfindlich ist.

4. Zellinie nach einem der Ansprüche 1 bis 3,
die bei der CNCM am 25. Februar 1991 unter der Nummer I-1045 sowie bei der Deutschen Sammlung von Mikroorganismen und Zellkulturen GmbH (DSM) am 14. Februar 1992 unter der Nummer DSM.ACC.2030 hinterlegt worden ist.

5. Zellinie nach Anspruch 1 oder Anspruch 2,
dadurch gekennzeichnet, daß sie gegen Retinsäure resistent ist.

6. Zellinie nach Anspruch 5,
wie sie durch Inkubation der Linie nach Anspruch 4 in Anwesenheit von all-trans-Retinsäure erhalten wird, gefolgt von der Isolierung der resistenten Zellen und der Amplifizierung der letzteren in ständiger Gegenwart von Retinsäure.

7. Zellkultur,
die im wesentlichen aus Zellen, die von einer Zellinie, wie der in einem der Ansprüche 1 bis 6 definierten Zellinie, abstammen, in Verbindung mit Nährstoffen, die die Proliferation der Zellen ermöglichen, besteht.

8. Verfahren zum Screening von Molekülen, die die Zellreifung wiederherstellen können,
dadurch gekennzeichnet, daß es das In-Kontakt-Bringen einer bestimmten Menge des untersuchten Moleküls mit einer Zellinie nach einem der Ansprüche 1 bis 4 umfaßt, gefolgt von dem Nachweis einer gegebenenfalls erfolgenden Differenzierung der Zellen dieser Linie.

9. Verfahren zum Screening von Molekülen, die die Resistenz aufheben können, die Zellen, und genauer Zellen, die für die akuten humanen promyelozytären Leukämien charakteristisch sind, gegenüber der Differenzierungswirkung der Retinsäure oder anderer Verbindungen, die normalerweise die Zellreifung induzieren können, aber hinsichtlich derer Resistenzerscheinungen der betroffenen Zellen beobachtet werden können, zeigen, wobei das Verfahren dadurch gekennzeichnet ist, daß es das In-Kontakt-Bringen einer bestimmten Menge des untersuchten Moleküls mit einer Zellinie nach Anspruch 5 oder Anspruch 6 umfaßt, gefolgt von dem Nachweis einer gegebenenfalls erfolgenden Differenzierung der Zellen dieser Linie in Gegenwart von Retinsäure oder der anderen obengenannten Verbindungen.

10. Verfahren nach Anspruch 8 oder Anspruch 9,
dadurch gekennzeichnet, daß der Nachweis der Differenzierung der Zellen ausgeführt wird:
- durch Nachweis der Expressionsprodukte spezifisch im Verlauf der Differenzierung exprimierter Gene mittels Antikörpern, die spezifisch die produzierten Polypeptide erkennen können, wobei diese Antikörper gegebenenfalls markiert sind, insbesondere radioaktiv oder enzymatisch, oder mittels Substraten, die gegebenenfalls markiert sind, insbesondere radioaktiv oder enzymatisch, wenn diese Expressionsprodukte enzymatisch aktiv sind,
- oder durch morphologische Untersuchung der Zellen
- oder ferner durch funktionale Untersuchung der Zellen.

11. Kit zum Ausführen eines Verfahrens nach einem der Ansprüche 8 bis 10,
dadurch gekennzeichnet, daß er enthält:
- eine Zellinie nach einem der Ansprüche 1 bis 6,
- geeignete Reagentien, um die Detektion der gegebenenfalls erfolgenden Differenzierung der Zellen zu ermöglichen, insbesondere:
-- Antikörper, die spezifisch die durch die Aktivierung der Expression bestimmter Gene produzierten Polypeptide erkennen können, wobei diese Antikörper gegebenenfalls markiert sind, insbesondere radioaktiv oder enzymatisch,
-- Substrate, die gegebenenfalls markiert sind, insbesondere radioaktiv oder enzymatisch, und mit denen die exprimierten, enzymatisch aktiven Produkte oder diejenigen, die nicht mehr exprimiert werden, reagieren können,
-- Enzyme, die gegebenenfalls markiert sind, insbesondere radioaktiv oder enzymatisch, und die mit den während der Zelldifferenzierung produzierten Substraten reagieren können,
-- Farbstoffe zum Anfärben der Zellen.

12. Verwendung einer Zellinie nach einem der Ansprüche 1 bis 6 als positives Kontrollsystem im Rahmen von Verfahren zur in vitro-Diagnose von akuten humanen promyelozytären Leukämien oder ferner von Verfahren zur in vitro-Diagnose oder ferner von Verfahren zur in vitro-Diagnose eines gegebenenfalls erfolgenden Auftretens hinsichtlich Retinsäure oder anderer Verbindungen, die die Zellreifung während der Behandlung eines Patienten mit diesen Verbindungen induzieren können, resistenter Zellen.

13. Kits zum Ausführen von Verfahren zur in vitro-Diagnose von akuten humanen promyelozytären Leukämien oder weiters von Verfahren zur in vitro-Diagnose des gegebenenfalls erfolgenden Auftretens hinsichtlich Retinsäure oder anderer Verbindungen, die die Zellreifung während der Behandlung eines Patienten mit diesen Verbindungen induzieren können, resistenter Zellen, wobei diese Kits eine Zellinie nach einem der Ansprüche 1 bis 6 als positives Kontrollsystem für das Vorliegen von Zellen, die für die akuten humanen promyelozytären Leukämien charakteristisch sind, und gegebenenfalls von hinsichtlich der obengenannten Verbindungen resistenten Zellen enthalten.

14. Verfahren zum Erhalten eines Wachstumsfaktors,
dadurch gekennzeichnet, daß es die folgenden Schritte umfaßt:
- Inkubation der Zellen der Linien nach einem der Ansprüche 1 bis 6 in einem Kulturmedium,
- Abnahme des Sekretionsmediums dieser Zellen und Nachweis des etwaigen Vorkommens eines oder mehrere Polypeptide, wie jener vorstehend definierten, mittels eines Proliferationstests und einer an hämatopoietischen Zellen eines Betugssystems oder der Linie nach Anspruch 4 oder der Linie nach Anspruch 4, die gegen Retinsäure resistent ist, oder an deren,Varianten erfolgten Differenzierung,
- biochemische Abtrennung des obengenannten Peptids oder der obengenannten Peptide.

## Claims

1. Permanent cell line, characterized in that it is essentially constituted by human promyelocyte cells, characteristic of acute promyelocyte leukemias, said cells being cytogenetically characterized by a translocation t(15;17), able to proliferate indefinitely in a synthetic culture medium containing fetal veal serum.

2. Cell line according to claim 1, characterized in that the cells constituting it have genetic imprints on electrophoresis gel of an identical nature, partly or totally, to the genetic imprint shown in fig. 1.

3. Cell line according to claim 1 or 2, characterized in that it is sensitive to retinoic acid.

4. Cell line according to one of the claims 1 to 3, filed at the CNCCM on 25.2.1991 under No. I-1045 and at the Deutsche Sammlung von Mikroorganismen und ZellKulturen GmbH (DSM) on 14.2.1992 under No. DSM.ACC.203D.

5. Cell line according to claim 1 or 2, characterized in that it is resistant to retinoic acid.

6. Cell line according to claim 5, as obtained by incubation of the line according to claim 4 in the presence of all-trans retinoic acid, followed by the isolation of resistant cells and the amplification thereof in the presence of retinoic acid.

7. Cell culture essentially constituted by cells belonging to a cell line as defined in one of the claims 1 to 6, in association with nutrient elements permitting the proliferation of the cells.

8. Process for screening molecules able to restore cell maturation, characterized in that it comprises contacting a given quantity of the studied molecule with a cell line according to one of the claims 1 to 4, followed by the revealing of a possible differentiation of the cells of said line.

9. Process for screening molecules able to remove the resistance of said cells and more particularly cells characteristic of human acute promyelocyte leukemias, with respect to the differentiation effect of retinoic acid or other compounds normally able to induce cell maturation, but for which resistance phenomena of the cells in question can be observed, said process being characterized in that it comprises the contacting of a given quantity of the studied molecule with a cell line according to claim 5 or 6, followed by the revealing of a possible differentiation of the cells of said line in the presence of retinoic acid or other aforementioned compounds.

10. Process according to claim 8 or 9, characterized in that the revealing of the differentiation of the cells is performed by the detection of expression products of genes specifically expressed during the differentiation, with the aid of antibodies able to specifically recognize the polypeptides produced, said antibodies being, if appropriate labelled, particularly radioactively or enzymatically, or with the aid of substrates, if appropriate labelled, particularly radioactively or enzymatically when said expression products are enzymatically active, or by a morphological study of the cells, or by a functional study of the cells.

11. Kit for performing a process according to one of the claims 8 to 10, characterized in that it comprises:
- a cell line according to one of the claims 1 to 6,
- appropriate reagents for permitting the detection of the possible differentiation of the cells, particularly:
antibodies able to specifically recognize the polypeptides produced by the activation of the expression of certain genes, said antibodies being, if appropriate, labelled in particular radioactively or enzymatically,
substrates, if appropriate labelled, particularly radioactively or enzymatically, and on which the enzymatically active products which are expressed or no longer expressed are liable to act,
enzymes, if appropriate labelled, particularly radioactively or enzymatically, and able to act on substrates produced during cell differentiation, cell dyes.

12. Use of a cell line according to one of the claims 1 to 6 as a positive control within the framework of in vitro diagnosis processes for human acute promyelocyte leukemias, or in vitro diagnosis processes
with respect to the possible appearance of cells resistant to retinoic acid or other compounds liable to induce cell maturation during the treatment of a patient with these compounds.

13. Kits for performing the aforementioned in vitro diagnosis processes of human acute promyelocyte leukemias, or in vitro diagnosis processes of the possible appearance of cells resistant to retinoic acid or other compounds liable to induce cell maturation during the treatment of a patient with said compounds, said kits comprising a cell line according to one of the claims 1 to 6 as a positive control of the presence of characteristic cells of human acute promyelocyte leukemias and, if appropriate, cells resistant to the aforementioned compounds.

14. Process for obtaining a growth factor, characterized in that it comprises the following stages:
- incubation of cells of lines according to any one of the claims 1 to 6 in a culture medium,
- sampling the secretion medium of these cells and detecting the possible presence of one or more polypeptides as defined hereinbefore with the aid of a proliferation and differentiation test performed on reference hematopoietic cells, or on the line according to claim 4 and the line according to claim 4 resistant to retinoic acid or variants thereof,
- biochemical separation of the aforementioned polypeptide or polypeptides.
